# EUROPÄISCHE PATENTSCHRIFT

(11) **EP 2 395 946 B1**
(45) Veröffentlichungstag und Bekanntmachung des Hinweises auf die Patenterteilung: **24.08.2016**
(21) Anmeldenummer: 10702491.1
(22) Anmeldetag: 04.02.2010
(51) Int. Cl.: A61F 2/34, A61F 2/00

(54) **KERAMISCHER PFANNENEINSATZ MIT INVERS-KONISCHEM FÜHRUNGSZAPFEN**
CERAMIC BALL SOCKET INSERT HAVING INVERSE-CONICAL GUIDE PINS
INSERT DE CAVITÉ CÉRAMIQUE À CHEVILLE DE GUIDAGE EN CÔNE INVERSÉ

(30) Priorität: 11.02.2009 DE 102009000771
(43) Veröffentlichungstag der Anmeldung: 21.12.2011
(73) Patentinhaber: CeramTec GmbH, 73207 Plochingen (DE)
(72) Erfinder: PREUSS, Roman, 73230 Kirchheim unter Teck (DE); PANDORF, Thomas, 73249 Wernau (DE); BLÄSKE, Yvonne, 71522 Backnang (DE); BÖGEL, Thomas, 73249 Wernau (DE)
(74) Vertreter: Scherzberg, Andreas Hans
(86) Internationale Anmeldenummer: PCT/EP2010/051371
(87) Internationale Veröffentlichungsnummer: WO 2010/091995

(56) Entgegenhaltungen:
- EP-A1- 0 482 320
- EP-A2- 1 297 800
- WO-A1-00/07526
- FR-A1- 2 661 605

## Beschreibung

Die Erfindung beschreibt eine Hüftgelenkprothese mit einem Pfanneneinsatz und einer Hüftpfanne, wobei auf dem Pol des Pfanneneinsatzes ein konischer Führungszapfen und am Grund der Hüftpfanne eine Führungsbohrung angeordnet sind und sich im Einbauzustand des Pfanneneinsatzes in der Hüftpfanne der Führungszapfen in der Führungsbohrung befindet.

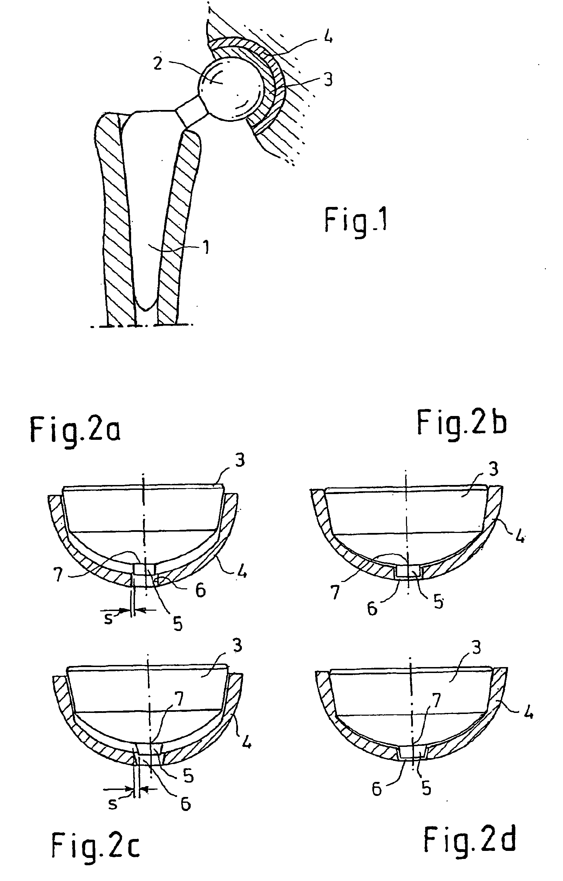

Im Markt existieren eine Vielzahl von Prothesen-Systemen zum Ersatz des natürlichen Hüftgelenkes. Diese bestehen in der Regel aus einem Schaft 1, gekoppelt mit einem Kugelkopf 2, und einer Hüftpfanne 4, gekoppelt mit einem Pfanneneinsatz 3, siehe Figur 1. Schaft 1 und Hüftpfanne 4 sind mit dem Körper durch Einwachsen in den Oberschenkel- bzw. Beckenknochen verbunden und sind Träger für den Kugelkopf 2 bzw. Pfanneneinsatz 3. Der Kugelkopf 2 ist in der Kalotte des Pfanneneinsatzes 3 drehbar gelagert - Freiheitsgrad: 1. Schaft 1 und Kugelkopf 2 werden dabei in der Regel durch konische Klemmung gekoppelt. Dies trifft zumeist auf Pfanneneinsatz 3 und Hüftpfanne 4 zu, die auch in der Regel durch konische Klemmung gekoppelt sind. Figur 1 zeigt eine Hüftprothese, bestehend aus Schaft 1, Kugelkopf 2, Hüftpfanne 3 und Pfanneneinsatz 4.

Während des Einsetzvorgangs insbesondere dünnwandiger Metallschalen in den Beckenknochen kann es zu Verformungen der Metallschalen im Bereich des Klemmkonus kommen. Dadurch wird das korrekte, funktionsgerechte Einsetzen des konisch geklemmten Pfanneneinsatzes erschwert. Im Extremfall tritt ein Verklemmen von Pfanneneinsatz und Hüftpfanne bei verkippter Lage des Pfanneneinsatzes in der Hüftpfanne auf. Die Verkippung des Pfanneneinsatzes ändert die Lastsituation und führt zu Punktlasten, welche sowohl die Dauerhaftigkeit der Klemmverbindung als auch die Lebensdauer des Pfanneneinsatzes an sich erheblich mindern können.

Weiterhin besteht gerade bei minimal-invasiven Operationstechniken in der Regel keine ausreichende Sicht auf das Operationsfeld, um zum Beispiel das korrekte Einsetzen des Pfanneneinsatzes in die Hüftpfanne unter visueller Kontrolle durchzuführen. Die konische Klemmung des Pfanneneinsatzes in der Hüftpfanne wirkt beim Einsetzen selbstzentrierend. Diese Führung des Pfanneneinsatzes beim Einsetzen in die Hüftpfanne wirkt aber nur bei geringen initialen Verkippungswinkeln. Tritt durch mangelnde Sicht des Operateurs eine stärkere initiale Verkippung auf, versagt die Selbstzentrierung und es tritt ein verkipptes Verklemmen mit den oben beschriebenen Folgen auf.

In der Vergangenheit wurde deshalb bei verschiedenen Pfanneneinsätzen eine zusätzliche Führung des Pfanneneinsatzes während der Einsetzbewegung realisiert. Zu diesem Zweck ist auf dem Pol des jeweiligen Pfanneneinsatzes 3 ein zylindrischer (siehe Figuren 2a, 2b) oder konischer Führungszapfen (siehe Figuren 2c, 2d) angeordnet. Der Führungszapfen wird beim Einsetzen des Pfanneneinsatzes 3 in eine entsprechend geformte Führungsbohrung 6 am Grund der Hüftpfanne 4 eingeführt und verhindert somit ein Verkippen des Pfanneneinsatzes 3. Die Figuren 2a, 2b, 2c, 2d zeigen einen Pfanneneinsatz 3 mit zylindrischem (oben) bzw. konischem (unten) Führungszapfen 5 am rückseitigen Pol. Die Spaltbreite s (siehe Figuren 2a, 2c) bestimmt, wie genau der Pfanneneinsatz 3 beim Einsetzen in die Hüftpfanne 4 geführt wird.

Aufgrund des geringen Bauraumes für Führungszapfen 5 und Führungsbohrung 6 ist die Führungslänge des Führungszapfens 5 in der Regel äußerst gering - insbesondere im Moment des Einsetzens des Pfanneneinsatzes, wenn die innerhalb der Führungsbohrung befindliche Länge des Führungszapfens noch gering, der Führungsbedarf für ein funktionsgerechtes Einsetzen des Pfanneneinsatzes allerdings am höchsten ist. Die Führungslänge steigt mit zunehmendem Einsetzen des Pfanneneinsatzes in die Hüftpfanne an, bei zunehmender Verbesserung der Führungswirkung. Allerdings nimmt dabei ebenfalls die Führungswirkung der konischen Klemmverbindung zwischen Pfanneneinsatz und Hüftpfanne zu, was die Notwendigkeit der Führung durch den Führungszapfen zunehmend mindert. Die Notwendigkeit der Führung und der Führungsgenauigkeit des Führungszapfens stehen somit in umgekehrtem Verhältnis.

Um ein Verkanten der Führung zu vermeiden, muss ein entsprechend großer Führungsspalt s realisiert werden. Weiterhin wird aufgrund der Durchmessertoleranzen von Führungszapfen und Führungsbohrung eine notwendige Mindestgröße des Führungsspaltes vorgegeben. Mit zunehmender Größe des Führungsspaltes sinkt allerdings die Führungsgenauigkeit zwischen Führungszapfen und Führungsbohrung und das Risiko der Verkippung des Pfanneneinsatzes in der Hüftpfanne steigt.

Der Erfindung liegt die Aufgabe zugrunde, eine Hüftgelenkprothese, einen Pfanneneinsatz und eine Hüftpfanne nach den Oberbegriffen der Ansprüche 1, 6 und 7 so weiterzubilden, dass ein funktionsgerechtes Einsetzen des Pfanneneinsatzes in die Hüftpfanne mit hoher Führungsgenauigkeit auch unter erschwerten Bedingungen ermöglicht wird.

Erfindungsgemäß wird diese Aufgabe durch die Merkmale der Ansprüche 1, 6 und 7 gelöst.

Erfindungsgemäß ist der Führungszapfen invers-konisch zulaufend ausgebildet, wobei an dem zum Pol gewandten Ende der Durchmesser des Führungszapfens geringer ist als an dem vom Pol abgewandten Ende des Führungszapfens.

Dadurch, dass auf dem Pol des Pfanneneinsatzes ein Führungszapfen ausgebildet ist, welcher invers-konisch zuläuft und welcher in eine zylindrische oder ebenfalls invers-konische Führungsbohrung im Hüftpfannengrund eingesetzt wird, wird die theoretische Führungslänge des Systems zu null und das Prinzip der Ausbildung einer klassischen Führung verlassen. Eine Führung und somit Unterstützung des Einsetzens des Pfanneneinsatzes zur Vermeidung der verkippten Lage erfolgt aber dennoch. Weiterhin sind extrem kleine Führungsspalte und somit eine hohe Führungsgenauigkeit realisierbar. Insbesondere wird gleich zu Beginn des Einsetzens des Pfanneneinsatzes in die Hüftpfanne der geringe Führungsspalt und damit die hohe Führungsgenauigkeit realisiert.

Wie dargestellt, zeichnet sich eine erfindungsgemässe Ausgestaltung dadurch aus, dass die Führungsbohrung invers-konisch zulaufend ausgebildet ist, wobei an dem zum Inneren bzw. Grund der Hüftpfanne gewandten Ende der Durchmesser der Führungsbohrung geringer ist als an dem vom Inneren abgewandten Ende der Führungsbohrung.

Eine andere erfindungsgemässe Ausgestaltung zeichnet sich dadurch aus, dass die Führungsbohrung zylindrisch ausgebildet ist.

Die Führungsbohrung kann auch aus bevorzugt zwei Abschnitten bestehen, wobei im ersten Abschnitt die Führungsbohrung zylindrisch und im zweiten Abschnitt invers-konisch zulaufend ausgebildet ist.

Vorteilhaft ist der erste Abschnitt an dem zum Inneren der Hüftpfanne gewandten Ende der Führungsbohrung angeordnet.

Ein erfindungsgemäßer Pfanneneinsatz für eine Hüftgelenkprothese mit einem am Pol angeordneten konischen Führungszapfen zur Einführung in eine entsprechende Führungsbohrung in einer Hüftpfanne ist in Anspruch 6 gezeigt.

Ein erfindungsgemäße Hüftpfanne für eine Hüftgelenkprothese mit einer am Grund der Hüftpfanne angeordneten konischen Führungsbohrung zur Aufnahme eines Führungszapfens eines Pfanneneinsatzes ist in Anspruch 7 dargestellt.

Durch die erfindungsgemäßen Ausbildungen des Pfanneneinsatz für eine Hüftgelenkprothese, der mit einem Führungszapfen versehen ist, welcher invers-konisch zulaufend gestaltet ist, ist ein verkipptes Einsetzen des Pfanneneinsatzes in die Hüftpfanne vermieden, da bereits zu Beginn des Einsetzvorgangs eine Führung bei der Einsetzbewegung des Pfanneneinsatzes erfolgt.

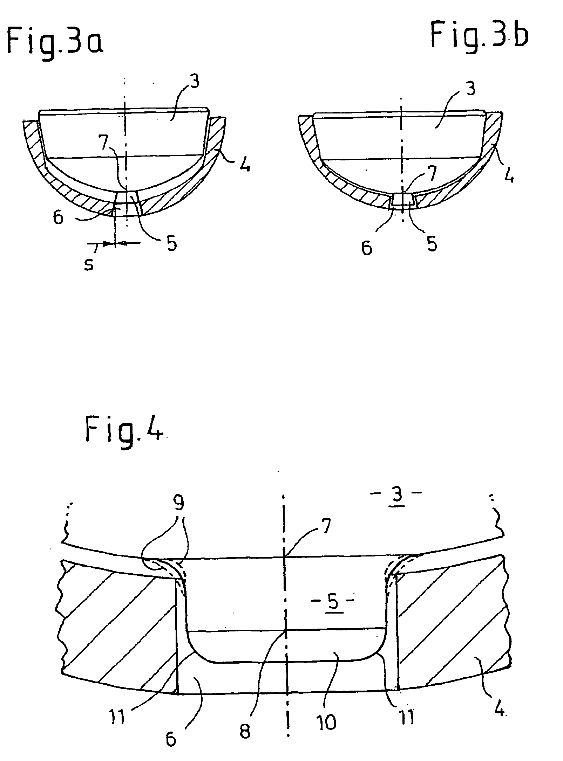

Bei zylindrischer Führungsbohrung bleibt der geringe Führungsspalt über die gesamte Länge der Führung konstant, während er bei invers-konischer Führungsbohrung mit zunehmender Einsetztiefe des Pfanneneinsatzes zunimmt. Die damit verbundene Abnahme der Führungsgenauigkeit entspricht allerdings auch der ebenfalls abnehmenden Notwendigkeit zur Führung, da die konische Klemmung zunehmend die Führungswirkung realisiert. Die Figuren 3a, 3b zeigen einen Pfanneneinsatz 3 mit invers-konischem Zapfen 5 am rückseitigen Pol 7. Die geringste Spaltbreite s führt zu hoher Führungswirkung bei geringem Verkantungsrisiko.

Ein weiterer positiver Effekt der invers-konischen Form des Zapfens 5 tritt bei Gestaltung von Verrundungsradien am Bauteil auf. Dies ist bei Verwendung spröder Werkstoffe notwendig, z.B. wenn der Pfanneneinsatz 3 erfindungsgemäss aus einem keramischen Werkstoff besteht. In solch einem Fall müssen die Kanten 9 des Zapfens 5 verrundet werden, um Kerbspannungen und Kantenausbrüche zu mindern.

Führt man an Verrundungsradien an zylindrischen oder konischen Zapfen Toleranzanalysen bzgl. größtem und kleinstem zulässigen Verrundungsradius durch, zeigen sich unter Umständen Kollisionen mit der Bohrung im Pfannenpol. Diese können nur durch Einschränkung von Toleranzen oder Reduktion des Zapfendurchmessers vermieden werden, siehe Figur 4. Toleranzeinschränkungen erhöhen in der Regel die Herstellkosten. Die Reduktion des Zapfendurchmessers erhöht die Spaltbreite s und mindert die Führungswirkung des Zapfens 5 beim Einsetzen des Pfanneneinsatzes. Figur 4 zeigt einen Pfanneneinsatz mit zylindrischem Zapfen 5 und verrundeten Kanten 9, Toleranzanalyse bzgl. größtem und kleinstem Kantenradius (gestrichelt) zeigt Kollision mit Hüftpfanne im Bereich der Durchgangsbohrung bei größtem Radius.

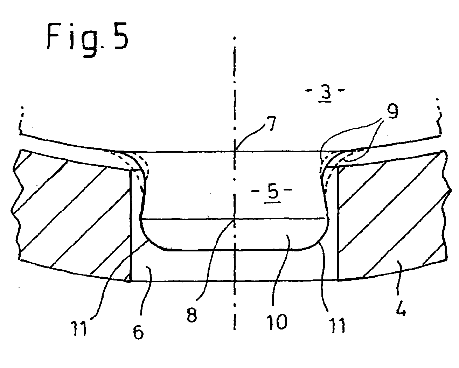

Bei invers-konischer Form des Zapfens 5 führt die Verjüngung des Zapfens zu einem vergrößerten Bauraum am einsatzseitigen Zapfenende. Toleranzanalysen der Kantenverrundung führen somit bei gleichen Werten zu einem größeren Abstand zur Durchgangsbohrung bzw. Führungsbohrung 6 der Hüftpfannen 4. Die Führungswirkung des Zapfens bleibt erhalten, ohne dass unerwünschte Kollisionen zwischen den Bauteilen auftreten, siehe Figur 5. Figur 5 zeigt einen Pfanneneinsatz mit invers-konischem Zapfen und verrundeten Kanten 9, Toleranzanalyse bzgl. größtem und kleinstem Kantenradius (gestrichelt) weist keine Kollision mit der Hüftpfanne auf. Zapfen und Führungszapfen sind zwei Begriffe, die jedoch denselben Gegenstand bezeichnen.

Der dem Pol 7 abgewandte Endbereich 10 des Führungszapfens 5 bzw. Zapfens ist abgerundet, d.h. der invers-konisch verlaufende Führungszapfen 5 weist einen abgerundeten Endbereich 10 auf (siehe Figuren 4 und 5). Hierdurch wird unter anderem das Einsetzen in die Führungsbohrung 6 erleichtert.

## Patentansprüche

1. Hüftgelenkprothese mit einem Pfanneneinsatz (3) und einer Hüftpfanne (4), wobei auf dem Pol (7) des Pfanneneinsatzes (3) ein Führungszapfen (5) und am Grund der Hüftpfanne (4) eine Öffnung angeordnet sind und sich im Einbauzustand des Pfanneneinsatzes (3) in der Hüftpfanne (4) der Führungszapfen (5) in der öffnung (6) befindet, **dadurch gekennzeichnet, dass** der Pfanneneinsatz (3) aus einer Keramik besteht, der Führungszapfen (5) invers-konisch zulaufend ausgebildet ist, wobei an dem zum Pol (7) gewandten Ende der Durchmesser des Führungszapfens (5) geringer ist als an dem vom Pol (7) abgewandten Ende (8) des Führungszapfens (5), dass der Führungszapten (5) verrundete kanten (9,11) aufweist, und dass die Öffnung (6) eine Führungsbohrung ist.

2. Hüftgelenkprothese nach Anspruch 1, **dadurch gekennzeichnet, dass** die Führungsbohrung (6) invers-konisch zulaufend ausgebildet ist, wobei an dem zum Inneren bzw. Grund der Hüftpfanne (4) gewandten Ende der Durchmesser der Führungsbohrung (6) geringer ist als an dem vom Inneren abgewandten Ende der Führungsbohrung (6).

3. Hüftgelenkprothese nach Anspruch 1, **dadurch gekennzeichnet, dass** die Führungsbohrung (6) zylindrisch ausgebildet ist.

4. Hüftgelenkprothese nach Anspruch 1 oder 2, **dadurch gekennzeichnet, dass** die Führungsbohrung (6) aus zwei Abschnitten besteht, wobei im ersten Abschnitt die Führungsbohrung (6) zylindrisch und im zweiten Abschnitt invers-konisch zulaufend ausgebildet ist.

5. Hüftgelenkprothese nach Anspruch 4, **dadurch gekennzeichnet, dass** der erste Abschnitt an dem zum Inneren der Hüftpfanne (4) gewandten Ende der Führungsbohrung (6) angeordnet ist.

6. Pfanneneinsatz (3) für eine Hüftgelenkprothese mit einem am Pol (7) angeordneten Führungszapfen (5) zur Einführung in eine entsprechende Führungsbohrung (6) in einer Hüftpfanne (4), **dadurch gekennzeichnet, dass** der Pfanneneinsatz (3) aus einer Keramik besteht und der Führungszapfen (5) invers-konisch zulaufend ausgebildet ist, wobei an dem zum Pol (7) gewandten Ende der Durchmesser des Führungszapfens (5) geringer ist als an dem vom Pol (7) abgewandten Ende des Führungszapfens (5), und dass der Führungszapfen (5) verrundete Kanten (9, 11) aufweist.

7. Hüftpfanne (4) für eine Hüftgelenkprothese mit einer am Grund der Hüftpfanne (4) angeordneten Öffnung zur Aufnahme eines Führungszapfens (5) eines Pfanneneinsatzes (3), **dadurch gekennzeichnet, dass** die Öffnung eine konische Führungsbohrung (6) ist, die Führungsbohrung (6) invers-konisch zulaufend ausgebildet ist, wobei an dem zum Inneren der Hüftpfanne (4) gewandten Ende der Durchmesser der Führungsbohrung (6) geringer ist als an dem vom Inneren abgewandten Ende der Führungsbohrung (6).

8. Hüftgelenkprothese nach einem der Ansprüche 1 bis 5, oder Pfanneneinsatz nach Anspruch 6, **dadurch gekennzeichnet, dass** der Führungszapfen (5) einen abgerundeten Endbereich (10) aufweist.

## Claims

1. A hip-joint prosthesis having a socket insert (3) and a hip socket (4), wherein arranged on the pole (7) of the socket insert (3) there is a guide pin (5) and at the base of the hip socket (4) there is an opening, and in the installed state of the socket insert (3) in the hip socket (4) the guide pin (5) is located in the opening (6), **characterised in that** the socket insert (3) consists of a ceramic material, the guide pin (5) is formed so that it tapers in an inverse-conical manner, wherein at the end facing the pole (7) the diameter of the guide pin (5) is smaller than at the end (8) of the guide pin (5) that faces away from the pole (7), **in that** the guide pin (5) has rounded edges (9, 11), and **in that** the opening (6) is a guide bore.

2. A hip-joint prosthesis according to claim 1, **characterised in that** the guide bore (6) is formed so that it tapers in an inverse-conical manner, wherein at the end facing the interior or base of the hip socket (4) the diameter of the guide bore (6) is smaller than at the end of the guide bore (6) that faces away from the interior.

3. A hip-joint prosthesis according to claim 1, **characterised in that** the guide bore (6) is formed so as to be cylindrical.

4. A hip-joint prosthesis according to claim 1 or 2, **characterised in that** the guide bore (6) consists of two sections, wherein in the first section the guide bore (6) is cylindrical and in the second section it is formed so that it tapers in an inverse-conical manner.

5. A hip-joint prosthesis according to claim 4, **characterised in that** the first section is arranged at the end of the guide bore (6) that faces the interior of the hip socket (4).

6. A socket insert (3) for a hip-joint prosthesis having a guide pin (5) arranged at the pole (7) for insertion into a corresponding guide bore (6) in a hip socket (4), **characterised in that** the socket insert (3) consists of a ceramic material, and the guide pin (5) is formed so that it tapers in an inverse-conical manner, wherein at the end facing the pole (7) the diameter of the guide pin (5) is smaller than at the end of the guide pin (5) that faces away from the pole (7), and **in that** the guide pin (5) has rounded edges (9, 11).

7. A hip socket (4) for a hip-joint prosthesis having an opening arranged at the base of the hip socket (4) to receive a guide pin (5) of a socket insert (3), **characterised in that** the opening is a conical guide bore (6), the guide bore (6) is formed so that it tapers in an inverse-conical manner, wherein at the end facing the interior of the hip socket (4) the diameter of the guide bore (6) is smaller than at the end of the guide bore (6) that faces away from the interior.

8. A hip-joint prosthesis according to one of claims 1 to 5, or a socket insert according to claim 6, **characterised in that** the guide pin (5) has a roundedoff end region (10).

## Revendications

1. Prothèse de l'articulation de la hanche, comprenant un insert cotyloïdien (3) et un cotyle (4), sachant qu'un tenon de guidage (5) est disposé sur le pôle (7) de l'insert cotyloïdien (3) et une ouverture est disposée dans le fond du cotyle (4) et, à l'état monté de l'insert cotyloïdien (3) dans le cotyle (4), le tenon de guidage (5) se trouve dans l'ouverture (6), **caractérisée en ce que** l'insert cotyloïdien (3) est constitué d'une céramique, le tenon de guidage (5) est réalisé sous une forme convergente inversement conique, sachant qu'à l'extrémité tournée vers le pôle (7), le diamètre du tenon de guidage (5) est plus faible qu'à l'extrémité (8) du tenon de guidage (5) qui est éloignée du pôle (7), **en ce que** le tenon de guidage (5) présente des arêtes (9, 11) arrondies, et **en ce que** l'ouverture (6) est un trou de guidage.

2. Prothèse de l'articulation de la hanche selon la revendication 1, **caractérisée en ce que** le trou de guidage (6) est réalisé sous une forme convergente inversement clonique, sachant qu'à l'extrémité tournée vers l'intérieur ou vers le fond du cotyle (4), le diamètre du trou de guidage (6) est plus faible qu'à l'extrémité du trou de guidage (6) qui est éloignée de l'intérieur.

3. Prothèse de l'articulation de la hanche selon la revendication 1, **caractérisée en ce que** le trou de guidage (6) est réalisé sous une forme cylindrique.

4. Prothèse de l'articulation de la hanche selon la revendication 1 ou 2, **caractérisée en ce que** le trou de guidage (6) est constitué de deux parties, sachant que dans la première partie, le trou de guidage (6) est réalisé sous une forme cylindrique et dans la deuxième partie sous une forme convergente inversement conique.

5. Prothèse de l'articulation de la hanche selon la revendication 4, **caractérisée en ce que** la première partie est disposée à l'extrémité du trou de guidage (6) qui est tournée vers l'intérieur du cotyle (4).

6. Insert cotyloïdien (3) pour une prothèse de l'articulation de la hanche, comprenant un tenon de guidage (5) disposé sur le pôle (7) et destiné à être engagé dans un trou de guidage (6) correspondant dans un cotyle (4), **caractérisé en ce que** l'insert cotyloïdien (3) est constitué d'une céramique et le tenon de guidage (5) est réalisé sous une forme convergente inversement conique, sachant qu'à l'extrémité tournée vers le pôle (7), le diamètre du tenon de guidage (5) est plus faible qu'à l'extrémité du tenon de guidage (5) qui est éloignée du pôle (7), et **en ce que** le tenon de guidage (5) présente des arêtes (9, 11) arrondies.

7. Cotyle (4) pour une prothèse de l'articulation de la hanche, comprenant une ouverture disposée dans le fond du cotyle (4) et destinée à recevoir un tenon de guidage (5) d'un insert cotyloïdien (3), **caractérisé en ce que** l'ouverture est un trou de guidage (6) conique, que le trou de guidage (6) est réalisé sous une forme convergence inversement conique, sachant qu'à l'extrémité tournée vers l'intérieur du cotyle (4), le diamètre du trou de guidage (6) est plus faible qu'à l'extrémité du trou de guidage (6) qui est éloignée de l'intérieur.

8. Prothèse de l'articulation de la hanche selon l'une des revendications 1 à 5, ou insert cotyloïdien (3) selon la revendication 6, **caractérisés en ce que** le tenon de guidage (5) présente une zone d'extrémité (10) arrondie.
